# EUROPÄISCHE PATENTSCHRIFT

(11) **EP 1 077 638 B1**
(45) Veröffentlichungstag und Bekanntmachung des Hinweises auf die Patenterteilung: **02.03.2005**
(21) Anmeldenummer: 99923553.4
(22) Anmeldetag: 07.05.1999
(51) Int. Cl.: A61B 5/03

(54) **EINFÜHRUNGSVORRICHTUNG FÜR GEHIRNSONDEN**
DEVICE FOR INTRODUCING BRAIN PROBES
DISPOSITIF D'INTRODUCTION POUR SONDES CEREBRALES

(30) Priorität: 09.05.1998 DE 19820808
(43) Veröffentlichungstag der Anmeldung: 28.02.2001
(73) Patentinhaber: GMS Gesellschaft für medizinische Sondentechnik mbH, 24247 Mielkendorf (DE)
(72) Erfinder: Fleckenstein, Wolfgang, 24247 Mielkendorf (DE)
(74) Vertreter: Schaefer, Konrad, Dipl.-Phys.
(86) Internationale Anmeldenummer: PCT/EP1999/003140
(87) Internationale Veröffentlichungsnummer: WO 1999/058053

(56) Entgegenhaltungen:
- WO-A-83/03190
- WO-A-97/42870
- DE-C- 19 502 183

## Beschreibung

Die Erfindung betrifft eine Vorrichtung der im Oberbegriff des Anspruches 1 genannten Art.

Meßsonden im Gehirn werden für die Forschung und insbesondere für die Intensivüberwachung bei Erkrankungen des Gehirns eingesetzt. insbesondere bei traumatisch bedingten Schwellungen des Gehirns. z.B. bei Schädelbruch. Mit solchen Messungen wird die bei Schwellungen stark verminderte Sauerstoffversorgung des Gehirns überwacht, um rechtzeitig operativ oder medikamentös eingreifen zu können.

Dabei ist die Überwachung mehrerer Parameter erforderlich. die sich in ihrer Aussage gegenseitig ergänzen und daher möglichst gleichzeitig oder kurz nacheinander bestimmt werden sollten. Solche Parameter sind z.B. der Druck im Gewebe, der Sauerstoffpartialdruck (pO₂), die mit Laser/Doppler-Verfahren bestimmbare Blutströmungsgeschwindigkeit sowie Fluoreszenzmessungen bestimmter Enzyme mit der NADH-Methode.

Zur Messung all dieser Parameter sind für das Gehirn geeignete Meßsonden mit distaler Meßstelle verfügbar, die dünn und flexibel sind, so daß sie auch bei längerfristiger Verlegung im Gehirngewebe dieses nicht schädigen.

Zur Einführung und Halterung der Sonden am Schädel sind gattungsgemäße Vorrichtungen im Gebrauch. Bei diesen kommt es auf sichere Halterung am Schädel und hochsterile Abdichtung der Öffnung zum Gehirn an. Dies insbesondere unter Berücksichtigung der Tatsache, daß Messungen häufig über Tage ausgeführt werden müssen. Dabei muß eine unsterile Umgebung und eine häufige Bewegung des Patienten berücksichtigt werden.

Eine gattungsgemäße Vorrichtung ist aus der
DE 195 02 183 C1
bekannt. Die hier verwendete, für solche Zwecke übliche Schädelschraube sichert eine hochfeste Lagerung der Sonden am Schädelknochen und eine sterile Abdichtung der Schädelbohrung. Mit der Quetschverschraubung wird die Sonde mit hoher Sterilität in der Längsbohrung der Schraube abgedichtet. Der Schutzschlauch sorgt für Zugentlastung der zumeist extrem zugempfindlichen Sonden und somit für deren Sicherung gegen Beschädigung bei Bewegungen des Patienten. Nachteilig bei dieser bekannten Konstruktion ist jedoch die Möglichkeit. nur eine Sonde zur Zeit verwenden zu können. Außerdem ist diese bekannte Konstruktion ihrer Natur nach nur für pO₂-Sonden geeignet. Andere Parameter müßten über gesondert verlegte Sonden ermittelt werden, was zusätzliche Schädelbohrungen erforderlich machen würde.

Eine besser geeignete gattungsgemäße Konstruktion ist aus dem
Katalog 93/94
der Firma GMS Gesellschaft für medizinische Sondentechnik mbH.
Dorfstraße 2, D-24247 Kiel-Mielkendorf.
Intracramal Insertion System. IIS
3 way system
bekannt. Die dort gezeigte Schädelschraube weist drei nebeneinander liegende Eingänge zu ihrer Längsbohrung auf. Durch diese Eingänge können gleichzeitig drei Sonden mit je eigener Quetschverschraubung verlegt werden. Es ist also die Messung von drei Parametern gleichzeitig möglich.

Nachteilig bei dieser Konstruktion ist die komplizierte Handhabung. Es müssen drei Quetschverschraubungen gesondert bedient werden, was die Gefahr der Undichtigkeit und somit Unsterilität bei einer der Verschraubungen erhöht. Die verwendete Schraube stellt eine teure Sonderkonstruktion dar. Es sind für jede Sonde eine getrennte Zugentlastung erforderlich. Dies führt zu hohen Kosten, die angesichts der üblichen Einmalverwendung das Krankenhaus stark belasten Ein wesentlicher Nachteil liegt auch darin, daß die Sonden unter unterschiedlichem Winkel durch die Längsbohrung in das Gehirn eintreten und dort im Winkel zueinander schräg aufgespreizt verlaufen. Wird bei einem Bedienungsfehler die Schädelschraube mit verlegten Sonden gedreht, so ergibt sich eine Verrührung des Gehirngewebes mit äußerst nachteiligen Folgen für den Patienten.

Eine nicht gattungsgemäße Konstruktion ist bekannt aus
WO 97/42870.

Die hieraus bekannte Einführungsvorrichtung besteht aus einem elastischen Stopfen mit mehreren Durchgangsbohrungen zur Durchführung von Sonden. Der Stopfen wird in die Schädelbohrung eingesetzt und läßt sich mit einer Schraubeinrichtung in Längsrichtung komprimieren. Dadurch ergibt sich sowohl eine Abdichtung gegenüber der Schädelbohrung als auch eine Abdichtung der durch den Stopfen geführten Sonden.

Nachteilig bei dieser Konstruktion ist zunächst die unsichere elastische Lagerung am Schädelknochen. Ferner sind sowohl die Abdichtungen des Stopfens gegenüber der Schädelbohrung als auch die Abdichtungen der Sonden im Stopfen sehr unsicher. Diese Konstruktion ließ sich daher nur in einer aufwendigen großen Operation verlegen, wobei anschließend an die Verlegung der Stopfen und dessen nach außen abführende Leitungen unter der Haut getunnelt angeordnet wurden.

Diese bekannte Konstruktion bietet daher den Vorteil der gleichzeitigen Verlegbarkeit mehrerer Sonden, hat aber den Nachteil, daß sie für den Routinebetrieb auf einer neurochirurgischen Intensivstation nicht brauchbar ist.

Die Aufgabe der vorliegenden Erfindung besteht darin, eine gattungsgemäße Vorrichtung zu schaffen, die billiger, einfacher und sicherer verlegbar ist.

Diese Aufgabe wird mit den Merkmalen des Anspruches 1 gelöst.

Bei der erfindungsgemäßen Konstruktion lassen sich mehrere Sonden durch mehrere Lumina desselben Führungsschlauches verlegen. Dabei ergeben sich zunächst Kostenvorteile. Es wird eine im wesentlichen konventionelle einfache Schädelschraube benötigt mit nur einer Quetschverschraubung. Der Führungsschlauch dient gleichzeitig zur Zugentlastung der Sonden und verbilligt weiter die Konstruktion. Die verwendete Schädelschraube gibt sicheren Halt am Schädelknochen und eine gute Abdichtung und Sterilität der Schädelbohrung. Die Sonden werden in dem Führungsschlauch mit der an sich bekannten Quetschverschraubung hochsteril abgedichtet. Es ist also hervorragende Sterilität gesichert. Da nur eine Schraubbetätigung zum Schließen der Quetschverschraubung benötigt wird. ergibt sich eine einfachere Verlegung und Handhabung der erfindungsgemäßen Konstruktion. Schließlich ergibt sich der Vorteil, daß die Sonden in den Lumina des Führungsschlauches parallel liegend geführt in das Gehimgewebe verlaufen. Bei unbeabsichtigter Verdrehung der Schädelschraube oder des Schutzschlauches kommt es daher nur zu geringen Gewebebeschädigungen im Gehirn. Insgesamt ist die erfindungsgemäße Konstruktion also erheblich billiger, einfacher und sicherer als die bekannten Konstruktionen.

Vorteilhaft sind die Merkmale des Anspruches 2 vorgesehen. Auf diese Weise wird sichergestellt, daß die sehr druckempfindlichen Sonden in dem Bereich, in dem sie die sehr harte Hirnhaut durchlaufen, vom Schutzschlauch geschützt sind.

Der Schutzschlauch könnte an seinem proximalen, außen liegenden Ende alle Sondenkupplungen nebeneinander liegend aufweisen. Vorteilhaft sind jedoch die Merkmale des Anspruches 3 vorgesehen. Auf diese Weise können die Sondenkupplungen an den Enden der Zweigschläuche einzeln ohne Behinderung durch die anderen Kupplungen betätigt werden. Außerdem können mit flexiblen Zweigschläuchen die Sondenabführungen in unterschiedliche Richtungen zu entsprechenden Anschlußgeräten verlegt werden.

Vorteilhaft sind die Merkmale des Anspruches 4 vorgesehen. Gegenüber der ebenfalls verwendbaren Ausbildung mit gesondertem Dichtring ergibt sich der Vorteil einer billigeren Konstruktion sowie einfacherer Handhabbarkeit, da der Dichtring nicht gesondert gehandhabt werden muß. Ferner ergibt sich eine definierte Längeneinstellung des Schutzschlauches z.B. für Zwecke des Anspruches 2.

Dabei sind vorteilhaft die Merkmale des Anspruches 5 vorgesehen. Angesichts der aus anderen Gründen, wie z.B. Gewebeverträglichkeit, Sterilisierbarkeit u. dgl. stark beschränkten Materialmöglichkeiten bei dem Führungsschlauch neigt der plastische Dichtring zum fließenden Nachgeben unter der Quetschkraft. Ein Federglied, z.B. in Form eines Gummiringes, kann langfristig die Quetschkraft aufrechterhalten.

Vorteilhaft sind die Merkmale des Anspruches 6 vorgesehen. Bei entsprechend standardisierten Längenabstimmungen für die einzelnen Sonden lassen sich diese sehr einfach auf die gewollten Verlegetiefen einstellen, ohne daß dazu Justieraufwand erforderlich wäre. Bei Ausbildung des Führungsschlauches gemäß Anspruch 3 können z.B. die Zweigschläuche in entsprechender Länge ausgebildet sein.

Wie bereits erwähnt. ist die Materialwahl beim Führungsschlauch durch die verschiedenen an ihn gestellten Anforderungen beschränkt. Geeignete Materialien sind zumeist von geringer Elastizität. Bei der Quetschung des Führungsschlauches. um mit diesem im Quetschbereich die Sonden sicher abzudichten, muß das Material des Führungsschlauches im Quetschbereich komprimiert werden. Das gelingt auch bei den für die sonstigen Anforderungen geeigneten Materialien. Probleme gibt es aber beim Lösen der Quetschverbindung. wenn bei mangelnder Rückstellkraft der Führungsschlauch sich nicht mehr von den Sonden löst und diese nicht gezogen werden können. Es muß dann der gesamte Führungsschlauch mit allen Sonden entfernt und neu verlegt werden. Zur Lösung dieser Probleme sind vorteilhaft die Merkmale des Anspruches 7 vorgesehen. Hierbei ist der Führungsschlauch im Bereich der Quetschdichtung aus anderem Material hoher Elastizität und insbesondere hoher Rückstellkraft ausgebildet. Es wird dadurch unter allen Umständen eine sichere Quetschdichtung der Sonden gewährleistet und nach Lösen der Quetschverbindung ein völliges Lösen der Sonden. so daß diese einzeln leicht gewechselt werden können. Die übrigen Teile des Führungsschlauches, also seine beiden Teile proximal und distal des Quetschstückes. können ohne Beachtung der Elastizitätseigenschaften in ihrer Materialwahl besser auf die gewünschten Anforderungen abgestimmt werden, also beispielsweise auf Gewebeverträglichkeit. gute Sterilisierbarkeit, Zugfestigkeit u. dgl.

Das Quetschstück kann in bekannter Weise von außen mit einem elastischen Quetschring gequetscht werden. Vorteilhaft sind jedoch die Merkmale des Anspruches 8 vorgesehen. Die Quetschverschraubung preßt die Flansche in Achsrichtung aufeinander zu und staucht somit das Quetschstück in axialer Richtung, wodurch es die erforderliche radiale Quetschdichtung der Sonden durchführt. Dadurch wird die Konstruktion sehr einfach und sicher.

Das Quetschstück kann mit den angrenzenden Enden des proximalen und distalen Teiles des Führungsschlauches zu einer Montageeinheit verbunden sein, beispielsweise durch Kleben und Schweißen oder durch geeignete Formschlußverbindung. Bei Kunststoffen sind solche Verbindungen jedoch schwierig herzustellen, insbesondere da sich die Klebverbindungen aus Gründen der Gewebe-Verträglichkeit zumeist verbieten. Vorteilhaft sind daher die Merkmale des Anspruches 9 vorgesehen. Durch völlig getrennte Ausbildung des Quetschstückes werden die genannten Probleme vermieden und wird die Konstruktion vereinfacht.

Ein Führungsschlauch mit völlig getrenntem Quetschstück läßt sich schwierig in der erfindungsgemäßen Vorrichtung montieren, da der aus distalem Teil, proximalem Teil und Quetschstück bestehende Fühmngsschlauch bei der Montage mit drei Händen gehalten werden müßte. Vorteilhaft sind daher die Merkmale des Anspruches 10 vorgesehen. Hierdurch wird eine einfach handhabbare Montageeinheit aller dreier Teile des Führungsschlauches geschaffen, wobei als Sicherungsstift beispielsweise ein in einem der Lumina verlegter Mandrin oder Führungsdraht verwendbar ist oder auch ein spezieller Sicherungsstift. der nur zwischen den an das Quetschstück angrenzenden Enden des proximalen und distalen Teiles des Führungsschlauches verlegt ist.

In der Zeichnung ist die Erfindung beispielsweise und schematisch dargestellt. Es zeigen:
- Fig. 1: einen Längsschnitt der erfindungsgemäßen Vorrichtung,
- Fig. 2: einen Schnitt nach Linie 2 - 2 in Fig. 1 und
- Fig. 3: einen Schnitt entsprechend Fig. 1 durch eine Ausführungsvariante der Vorrichtung mit Quetschstück.

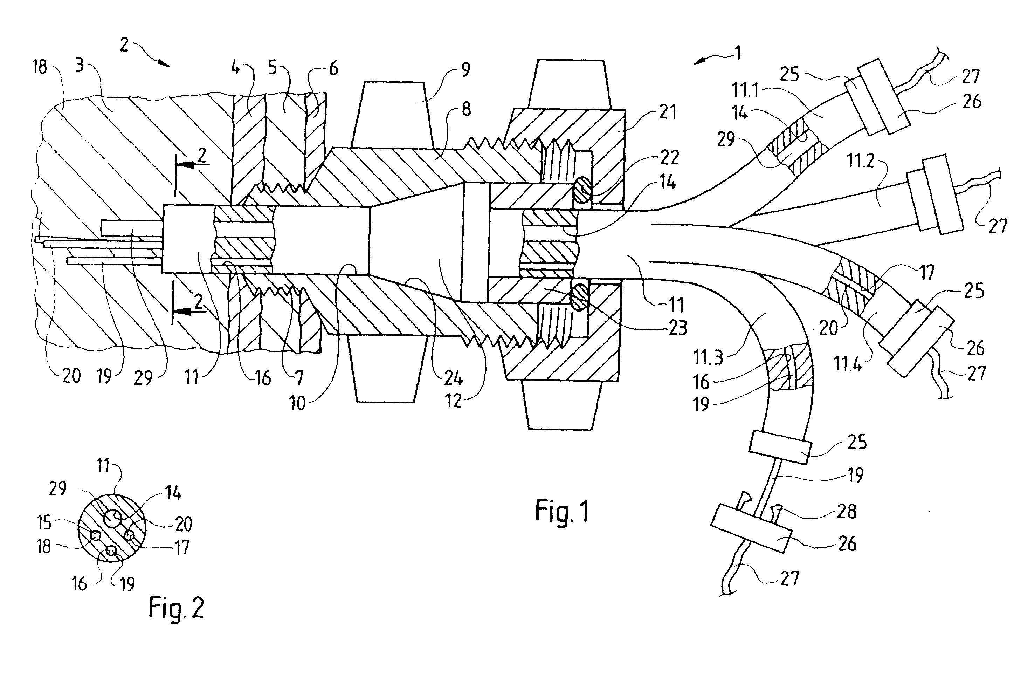

In Fig. 1 ist eine Einführungsvorrichtung 1 in Verlegestellung am Schädel 2 eines Patienten dargestellt. Dieser zeigt im Schnitt das Gehirngewebe 3, die Gehirnhaut 4, den Schadelknochen 5 und die Kopfhaut 6.

In eine zuvor durch den Schädelknochen 5 gesetzte Schädelbohrung ist das distale Gewindestück 7 einer Schädelschraube 8 eingeschraubt unter Abdichtung auf der Kopfhaut 6 mit entsprechenden Konusflächen der Schädelschraube. Als Einschraubhilfe dienen Griffstücke 9 an der Schädelschraube.

Durch eine Längsbohrung 10, die die Schädelschraube 8 durchsetzt, ist ein Führungsschlauch 11 verlegt, der an einer definierten Stelle seiner Länge einen integral mit dem Material des Führungsschlauches 11 ausgebildeten Dichtung 12 trägt, der im Ausführungsbeispiel konusförmig ausgebildet ist.

Der Führungsschlauch 11 ist, wie auch Fig. 2 zeigt, massiv aus geeignetem flexiblem Material ausgebildet. in dem mehrere, im Ausführungsbeispiel 4 längsverlaufende Kanäle ausgebildet sind, die im folgenden als Lumina bezeichnet werden.

Die Lumina dienen zum Durchführen langer flexibler Meßsonden und können unterschiedliche Innendurchmesser aufweisen. Ein größeres Lumen 14 dient zur Durchführung einer dickeren Meßsonde 29, während drei engere Lumina 15, 16 und 17 der Durchführung dreier dünnerer Meßsonden 18, 19 und 20 dienen.

Am proximalen Ende der Längsbohrung 10 weist die Schädelschraube 8 eine Quetschverschraubung auf, gebildet durch eine mit Innengewinde auf das proximale Ende der Schädelschraube 8 schraubbare Quetschmutter 21, die über einen federelastischen. z.B. aus geeignetem Gummi ausgebildeten Ring 22 eine Schiebehülse 23 belastet, welche gegen die Stirnfläche des konischen Dichtringes 12 anliegt und diesen gegen einen Innenkonus 24 der Schädelschraube 8 komprimiert.

Beim Anziehen der Quetschmutter 21 wird der Dichtring 12 gegen den Innenkonus 24 derart komprimiert, daß er radial nach innen verformt unter dichtender Verquetschung der Meßsonden 18, 19 und 20 in ihren jeweiligen Lumina 15, 16 und 17 sowie unter äußerer Abdichtung des Führungsschlauches 11 gegen die Schädelschraube 8. Es ist dafür Sorge zu tragen, daß zur Gewährleistung einer sicheren Quetschung die verlegten Meßsonden in ihrem Außendurchmesser auf dem Innendurchmesser des jeweiligen Lumens passend abgestimmt sind. Wird in einem Lumen keine Sonde benötigt, so ist wenigstens im Bereich der Quetschdichtung ein Füllstab vorzusehen.

Proximal außerhalb der Schädelschraube 8 verzweigt der Führungsschlauch 11 bei der dargestellten Ausführungsform in mehrere Zweigschläuche 11.1, 11.2, 11.3 und 11.4. Jedes der Lumina des Führungsschlauches 11 verläuft in einem der Zweigschläuche weiter, so z.B. das Lumen 14 im Zweigschlauch 11.1 und das Lumen 17 im Zweigschlauch 11.4.

Am proximalen Ende eines jeden der Zweigschläuche 11.1 bis 11.4 ist eine Sondenkupplung vorgesehen. bestehend jeweils aus einem zweigschlauchseitigen Kupplungsstück 25 und einem sondenseitigen Kupplungsstück 26. Das sondenseitige Kupplungsstück 26 trägt distal eine der Meßsonden 29, 18, 19 oder 20 und proximal eine Leitung 27, die zu einem nicht dargestellten Meßgerät führt und die beispielsweise als elektrische. optische oder hydraulische Signalübertragungsleitung ausgebildet sein kann, je nach verwendetem Sondentyp.

Bei dem Zweigschlauch 11.3 sind die Kupplungsstücke 25 und 26 auseinandergezogen dargestellt. Die Meßsonde 19 ist also ein Stück weit aus dem Führungsschlauch 11 herausgezogen Es ist hier beispielsweise ein Paar Klammern 28 dargestellt. das zur einrastenden Kupplungsverbindung zwischen den Kupplungsstucken 25 und 26 dienen kann.

In nicht dargestellter Ausführung kann der Führungsschlauch 11 auch unverzweigt bis zu seinem proximalen Ende fühlen. an dem die Kupplungen für die verschiedenen Sonden z.B. auf einem gemeinsamen Kupplungskörper vorgesehen sein können.

Die Zweigschläuche 11.1 bis 11.4 können unterschiedlich lang ausgebildet sein. Sie können auf die jeweils einzuführenden Sonden in ihrer Länge derart abgestimmt sein, daß die Meßsonden mit abgestufter Verlegetiefe in das Gehirn ragen. Auf diese Weise kann eine gewünschte Anordnung der am distalen Ende liegenden Meßstellen der Sonden erreicht werden.

Da der Dichtring 12 integral, also in definierter Längenstellung auf dem Führungsschlauch 11 befestigt ist, wird der Führungsschlauch 11 nach Herstellung der Quetschverbindung durch die Schädelschraube 8 in ihrer Befestigungslage im Schädelknochen 5 in definierter Längenstellung gegenüber dem Gehirn gehalten. Dadurch wird auf einfache Weise die gewünschte Tiefenstellung der Meßsonden gewährleistet.

Es wird dabei auch, wie in Fig. 1 dargestellt, bei entsprechender Länge des distal vom Dichtring 12 liegenden Endstückes des Führungsschlauches 11 sichergestellt, daß dieser die Gehirnhaut 4 durchragt und dort die empfindlichen Meßsonden gegen die hohen Krafteinwirkungen der Gehirnhaut 4 schützt.

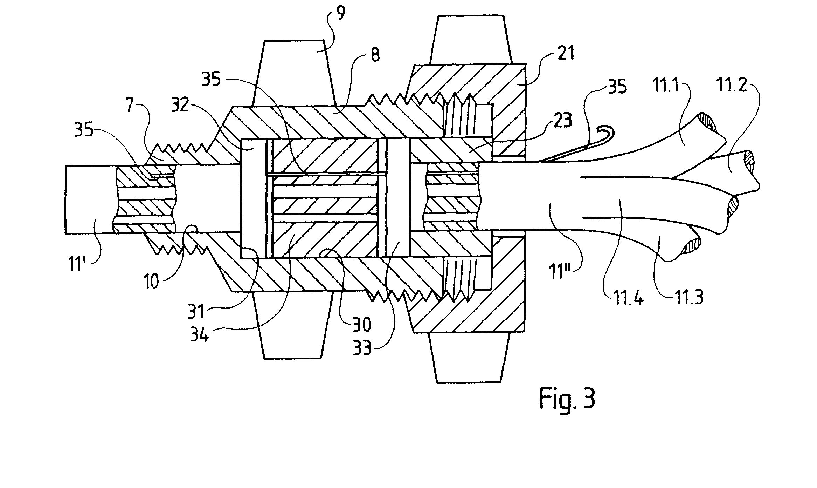

Fig. 3 zeigt eine Ausführungsvariante der in Fig. 1 dargestellten Konstruktion. Soweit möglich, werden dieselben Bezugszeichen verwendet. Der in Fig. 1 dargestellte Schadel 2 des Patienten und die dort dargestellten Sonden 18, 19, 20 und 29 sind zur zeichnerischen Vereinfachung weggelassen.

Die Schädelschraube 8 und die Quetschmutter 21 ebenso wie die Schiebehülse 23 entsprechen der Konstruktion der Fig. 1. Der elastische Ring 22 der Fig. 1 ist weggelassen, da er hier nicht erforderlich ist. Anstelle des Innenkonus 24 ist in der Schädelschraube 8 unmittelbar proximal anschließend an deren Längsbohrung 10 eine etwas größere Bohrung 30 vorgesehen, in die die Schiebehülse 23, wie dargestellt. greift. Die Bohrung 30 grenzt mit einer Stufe 31 an die Längsbohrung 10.

Der in der Ausführungsform der Fig. 1 einstückige Führungsschlauch 11 ist bei der Ausführungsform der Fig. 3 in ein distales Stück 11' und ein proximales Stück 11" unterteilt. Das proximale Ende des distalen Teiles 11' weist einen Auβenflansch 32 und das distale Ende des proximalen Teiles 11" einen Außenflansch 33 auf. Die beiden Außenflansche 32 und 33 sind integral mit den Teilen 11' und 11" des Führungsschlauches ausgebildet und passen mit ihrem Außendurchmesser in die Bohrung 30. Bei Betätigung der Quetschverschraubung, also Anziehen der Quetschmutter 21 gegenüber der Schädelschraube 8 werden somit die beiden Außenflansche 32 und 33 in Richtung aufeinander zu bewegt.

Zwischen den beiden Außenflanschen 32 und 33 ist ein Quetschstück 34 angeordnet. das, wie Fig. 3 zeigt, getrennt von den Teilen 11' und 11" des Führungsschlauches ausgebildet ist. Es besteht aus anderem Material als die Teile des Führungsschlauches, und zwar aus hochelastischem Material mit guter Federwirkung und insbesondere hoher Rückstellkraft.

Das Quetschstück 34 entspricht in seinem Außendurchmesser dem Innendurchmesser der Bohrung 30 und weist, wie dargestellt. dieselben Durchgangskanäle, also Lumina 14, 15, 16 und 17 auf, wie die Teile 11' und 11" des Führungsschlauches, die in der Ausführungsform der Fig. 3 genauso ausgebildet sind wie bei der Ausführungsform der Fig. 1.

Werden die Teile 11' und 11" des Führungsschlauches sowie das Quetschstück 34, wie in Fig. 3 dargestellt, angeordnet und werden die Sonden, wie in Fig. 1 dargestellt, eingesteckt, so kann anschließend die Quetschmutter 21 angezogen werden. Dadurch werden die Außenflansche 32 und 33 in axialer Richtung aufeinander zu bewegt, kommen in Anlage an die axialen Enden des Quetschstückes 34 und quetschen dieses in axialer Richtung. Das Quetschstück 34 kommt außen in Anlage an die Bohrung 30 und quetscht somit radial nach innen auf die in seinen Lumina verlegten Sonden unter Schaffung einer hochsterilen Abdichtung.

Nach Lösen der Quetschverschraubung stellt sich das aus hochelastischem Material mit guter Rückstellkraft bestehende Quetschstück 34 unter Zurückschieben des proximalen Teiles 11" des Führungsschlauches wieder in seine Ausgangsform zurück unter Aufweitung seiner Lumina und somit unter völliger Freigabe der darin liegenden Sonden, die leicht herausgezogen und beispielsweise gewechselt werden können.

Zur Sicherung der in Fig. 3 dargestellten Montagelage der beiden Teile 11' und 11" des Führungsschlauches und des Quetschstückes 34 zueinander, wobei die Außenflansche 32, 33 und das Quetschstück 34 konzentrisch liegen müssen und die Lumina fluchtend verlaufen müssen, ist ein Sicherungsstift 35 vorgesehen, der achsparallel in den Teilen 11' und 11" des Führungsschlauches und im Quetschstück 34 verlegt ist. Im Ausführungsbeispiel ist er im Teil 11' z.B. durch Verschweißung befestigt. Im Quetschstück 34 und im Teil 11" des Führungsschlauches ist er jedoch längsverschiebbar gelagert. Er ragt proximal, wie in Fig. 3 dargestellt, seitlich aus dem Teil 11" des Führungsschlauches heraus und ist dort zu einem Haken abgebogen. Der Sicherungsstift 35 sichert die drei Teile des Führungsschlauches aneinander, läßt jedoch ihre Langsverschiebbarkeit zur Herstellung und Lösung der Quetschverbindung zu.

In anderer Ausführungsform kann der Sicherungsstift 35 weggelassen sein. Die Sicherung der Teile des Führungsschlauches und des Quetschstückes, die bei der Montage des Führungsschlauches hilfreich ist, kann durch Führungsdrähte erfolgen, die anstelle der Sonden durch Lumina des Führungsschlauches gesteckt sind. Alternativ kann das Quetschstück 34 auch. mit den Teilstücken 11' und 11" des Führungsschlauches fest verbunden sein, z.B. durch Verklebung oder Verschweißung der Endflächen oder durch Herstellung einer sichernden Kupplungsverbindung über geeignete Formschlußausbildung der Endflächen.

## Patentansprüche

1. Vorrichtung (1) zum Einführen einer dünnen flexiblen Meßsonde (29) in das Gehirngewebe (3), mit einer Schädelschraube (8), die in einer Längsbohrung (10) von der Meßsonde (29) und einem diese in einem Lumen (14) aufnehmenden Führungsschlauch (11, 11', 11") durchsetzt wird, mit einer Quetschverschraubung (12, 23, 21) zur radial quetschenden Abdichtung der Meßsonde (29) und des Führungsschlauches an der Schädelschraube (8) und mit einem Schutzschlauch, der die Meßsonde umgibt und die Schädelschraube mit einer Sondenkupplung (25, 26) verbindet, **dadurch gekennzeichnet, daß der** Führungsschlauch (11, 11', 11") weitere getrennte Lumina (15, 16, 17) **zur** Aufnahme weiterer Meßsonden (18, 19, 20) aufweist, den Schutzschlauch ausbildet und an den proximalen Enden der Lumina je eine Sondenkupplung (25, 26) trägt.

2. Vorrichtung nach Anspruch 1, **dadurch gekennzeichnet, daß** der Führungsschlauch (11, 11', 11") eine definierte Quetschstelle (12, 34) aufweist, von der ab seine distale Länge derart ist, daß er in Verlegestellung die Hirnhaut (4) durchragt.

3. Vorrichtung nach Anspruch 1, **dadurch gekennzeichnet, daß** der Führungsschlauch (11, 11', 11") in Verlegestellung proximal außerhalb der Schädelschraube (8) in Zweigstücke (11,1 - 11,4) mit je einem Lumen (14, 15, 16, 17) verzweigt.

4. Vorrichtung nach Anspruch 1, mit einem den Führungsschlauch (11) umgebenden plastischen Dichtring (12), **dadurch gekennzeichnet, daß** der Dichtring (12) integral mit dem Führungsschlauch ( 11) ausgebildet ist.

5. Vorrichtung nach Anspruch 4, **dadurch gekennzeichnet, daß** die Quetschverschraubung (23, 21) ein vorspannbares Federglied (22) aufweist.

6. Vorrichtung nach Anspruch 1, **dadurch gekennzeichnet, daß** die Längen der Meßsonden (29, 18, 19, 20) und der Lumina (14, 15, 16, 17) derart aufeinander abgestimmt sind, daß sich eine definierte Abstufung der Verlegetiefen der distalen Enden der Meßsonden (29, 18, 19, 20) ergibt.

7. Vorrichtung nach Anspruch 1, **dadurch gekennzeichnet, daß** der Führungsschlauch (11, 11', 11") über einen Teil seiner Länge im Bereich der Quetschverschraubung als Quetschstück (34) aus anderem Material höherer Elastizität und Rückstellkraft ausgebildet ist.

8. Vorrichtung nach Anspruch 7, **dadurch gekennzeichnet, daß** die an das Quetschstück (34) angrenzenden Enden des distalen (11') und proximalen (11") Teiles des Führungsschlauches mit integralen Außenflanschen (32, 33) zum axialen Quetschangriff der Schraubverbindung (8, 21) ausgebildet sind.

9. Vorrichtung nach Anspruch 8, **dadurch gekennzeichnet, daß** das Quetschstück (34) getrennt von dem distalen (11') und proximalen (11") Teil des Führungsschlauches ausgebildet ist.

10. Vorrichtung nach Anspruch 9, **dadurch gekennzeichnet, daß** das distale (11') und das proximale (11") Teil des Führungsschlauches durch das Quetschstück (34) hindurch mit einem Sicherungsstift (35) verbunden sind

## Claims

1. Device (1) for insertion of a thin flexible measuring probe (29) into the brain tissue, with a skull screw (8) which has passing through it in a longitudinal bore (10) the measuring probe (29) and a guide hose (11, 11', 11") which receives the measuring probe in a lumen (14), with a compressive screw connection (12, 23, 21) for radially compressive sealing of the measuring probe (29) and of the guide hose on the skull screw (8), and with a protective sleeve which surrounds the measuring probe and connects the skull screw to a probe coupling (25, 26), **characterised in that** the guide hose (11, 11', 11") has further separate lumina (15, 16, 17) to receive further measuring probes (18, 19, 20), forms the protective hose and bears a probe coupling (25, 260) on the proximal end of each lumen.

2. Device as claimed in Claim 1, **characterised in that** the guide hose (11, 11', 11") has a defined compression site (12, 34) from which its distal length is such that in the installed position it projects through the scalp (4).

3. Device as claimed in Claim 1, **characterised in that** in the installed position the guide hose (11, 11', 11") branches proximally outside the skull screw (8) into branch parts (11. 1 - 11.4) each having a lumen (14, 15, 16, 17).

4. Device as claimed in Claim 1, with a plastic sealing ring (12) surrounding the guide hose (11), **characterised in that** the sealing ring (12) is integral with the guide hose (11).

5. Device as claimed in Claim 4, characterise din that the compressive screw connection (23, 21) has a spring element (22) which can be initially tensioned.

6. Device as claimed in Claim 1, **characterised in that** the lengths of the measuring probes (29, 18, 19, 20) and of the lumina (14, 15, 16, 17) are tailored to one other in such a way as to produce a defined graduation of the depths of installation of the distal ends of the measuring probes (29, 18, 19, 20).

7. Device as claimed in Claim 1, **characterised in that** the guide hose (11, 11', 11") is constructed over a part of its length in the region of the compressive screw connection as a compression piece (34) which is made from different material with a higher resilience and restoring force.

8. Device as claimed in Claim 7, **characterised in that** the ends of the distal (11') and proximal (11") parts of the guide hose adjoining the compression piece are constructed with integral outer flanges (32, 33) for axial compression of the screw connection (8,21).

9. Device as claimed in Claim 8, **characterised in that** the compression piece (34) is constructed separately from the distal (11') and proximal (11") parts of the guide hose.

10. Device as claimed in Claim 9, **characterised in that** the distal (11') and the proximal (11") part of the guide hose are connected through the compression piece (34) by a securing pin (35).

## Revendications

1. Dispositif (1) d'introduction d'une fine sonde de mesure (29) flexible dans les tissus cérébraux (3), doté d'une vis crânienne (8) traversée dans un alésage longitudinal (10) par la sonde de mesure (29) et un tuyau de guidage (11, 11', 11 ") dans lequel la sonde (29) est logée dans une lumière (14), d'un raccord à compression (12, 23, 21) destinée à l'étanchéification, dans la vis crânienne (8), de la sonde de mesure (29) et du tuyau de guidage par serrage radial, et doté d'un tuyau de protection entourant la sonde de mesure et reliant la vis crânienne à un dispositif d'accouplement (25, 26) de la sonde, **caractérisé en ce que** le tuyau de guidage (11, 11', 11") présente des lumières supplémentaires (15, 16, 17) destinées à recevoir d'autres sondes de mesure (18, 19, 20), forme le tuyau de protection et porte un accouplement (25, 26) pour sonde à l'extrémité proximale de chaque lumière.

2. Dispositif selon la revendication 1, **caractérisé en ce que** le tuyau de guidage (11, 11', 11") présente une section de compression définie (12, 34) à partir de laquelle sa longueur distale est telle que, en position de pose, il traverse la méninge (4).

3. Dispositif selon la revendication 1, **caractérisé en ce que** le tuyau de guidage (11, 11', 11") présente, en position de pose, du côté proximal à l'extérieur de la vis crânienne (8) une ramification formant des embranchements (11.1 - 11.4) présentant chacun une lumière (14, 15, 16, 17).

4. Dispositif selon la revendication 1 doté d'une bague d'étanchéité plastique (12) entourant le tuyau de guidage (11), **caractérisé en ce que** la bague d'étanchéité (12) fait intégralement corps avec le tuyau de guidage (11).

5. Dispositif selon la revendication 4, **caractérisé en ce que** le raccord à compression (23, 21) présente un élément ressort (bague 22) à précontrainte.

6. Dispositif selon la revendication 1, **caractérisé en ce que** les longueurs des sondes de mesure (29, 18, 19, 20) et des lumières (14, 15, 16, 17) snot adaptées les unes aux autres de façon à ce qu'il en résulte un échelonnement défini de la profondeur de pose des extrémités distales des sondes de mesure (29, 18, 19, 20).

7. Dispositif selon la revendication 1, **caractérisé en ce que** le tuyau de guidage (11; 11', 11"), sur une partie de sa longueur au niveau du joint à compression, forme une section de compression (34) réalisée en un autre matériau d'élasticité et de force de rappel supérieures.

8. Dispositif selon la revendication 7, **caractérisé en ce que** les extrémités de la partie distale (11') et de la partie proximale (11") du tuyau de guidage contiguës à la section de compression (34) forment des collerettes extérieures intégrales (32, 33) destinées à assurer la compression axiale avec le raccord à vis (8, 21).

9. Dispositif selon la revendication 8, **caractérisé en ce que** la section de compression (34) forme une pièce séparée de la partie distale (11') et de la partie proximale (11") du tuyau de guidage.

10. Dispositif selon la revendication 9, **caractérisé en ce que** la partie distale (11') et la partie proximale (11") du tuyau de guidage sont reliées l'une à l'autre par une broche de sécurité (35) traversant la section de compression (34).
